# EUROPEAN PATENT SPECIFICATION

(11) **EP 1 592 417 B1**
(45) Date of publication and mention of the grant of the patent: **18.04.2007**
(21) Application number: 04704703.0
(22) Date of filing: 23.01.2004
(51) Int. Cl.: A61K 31/205, A61K 31/17, A61P 3/10

(54) **COMBINATION OF ANTIDIABETIC DRUGS**
KOMBINATION VON ARZNEIMITTELN GEGEN DIABETES
COMBINAISON DE MEDICAMENTS ANTIDIABETIQUES

(30) Priority: 10.02.2003 IT RM20030053
(43) Date of publication of application: 09.11.2005
(73) Proprietor: SIGMA-TAU Industrie Farmaceutiche Riunite S.p.A., 00144 Roma (IT)
(72) Inventor: PESSOTTO, Pompeo, Sigma-Tau Ind.Farm.Riunite S.p.a, I-00040 Pomezia RM (IT); GIANNESSI, Fabio, Sigma-Tau Ind.Farm.Riunite S.p.a, I-00040 Pomezia RM (IT)
(74) Representative: Spadaro, Marco
(86) International application number: PCT/IT2004/000014
(87) International publication number: WO 2004/069239

(56) References cited:
- WO-A-99/59957
- GIANNESSI, FABIO ET AL: "Discovery of a Long-Chain Carbamoyl Aminocarnitine Derivative, a Reversible Carnitine Palmitoyltransferase Inhibitor with Antiketotic and Antidiabetic Activity" JOURNAL OF MEDICINAL CHEMISTRY , 46(2), 303-309 CODEN: JMCMAR; ISSN: 0022-2623, 2003, XP002281384
- DAGOGO-JACK S ET AL: "PATHOPHYSIOLOGY OF TYPE 2 DIABETES AND MODES OF ACTION OF THERAPEUTIC INTERVENTIONS" ARCHIVES OF INTERNAL MEDICINE, AMERICAN MEDICAL ASSOCIATION, CHICAGO, IL, US, vol. 157, no. 16, 8 September 1997 (1997-09-08), pages 1802-1817, XP000872498 ISSN: 0003-9926

## Description

The invention disclosed herein relates to the preparation of medicaments, particularly for the treatment of diabetes, and more particularly to combinations of active ingredients with a synergic effect on this disease.

### Background to the invention

Diabetes is a widespread disease and, according to the WHO, in some cases has reached epidemic proportions.

It is currently the fourth most common cause of death in the industrialised countries and is rapidly increasing in the developing countries.

The estimated incidence of diabetes world-wide rose from 30 million patients in 1985 to 135 million in 1995, with forecasts of 240 million in 2001 and 300 million in 2025.

Various clinical forms of diabetes are known, the most common of which are type 1 and type 2 diabetes.

Type 1 diabetes is caused by the autoimmune destruction of the pancreatic islets, leading to deficient insulin secretion. In Europe and North America, type 1 diabetes is the third most frequent chronic disease in juvenile patients.

Type 2 diabetes is widespread in Western countries, where it is more common in the elderly and obese population. The incidence of type 2 diabetes is estimated as being around 5% of the population, with peaks of 10-20% in some communities. The highest rates of increase of type 2 diabetes are to be found in the developing countries or their communities, where an increase in the standard of living is associated with the adoption of the Western life-style and socio-economic modernisation. In extreme conditions, the disease may affect more than 30% of the population.

Type 2 diabetes accounts for 85% of cases of diabetes. It is a multifactorial disorder with genetic and environmental components.

Progress in the understanding of the genetic causes of the disease is slow owing to its heterogeneous nature and to interaction with environmental factors. Nevertheless, analysis of the genes which are candidates as causing the disease constitutes a widely adopted approach and furnishes guidelines for research in this field.

Apart from age, the onset of the disease is facilitated by the type of diet, lack of physical exercise and, particularly, obesity, with which it is associated in 90% of cases.

In countries such as the United States, where 30% of the population are obese or overweight, the incidence of type 2 diabetes is 7%, whereas in Japan, where obesity is rare, an incidence of 1% has been estimated. The incidence is destined to rise in proportion to the lengthening of the duration of life and to the growth of affluence of the population.

Type 2 diabetes is characterised by hyperglycaemia, which is caused by a deficiency of insulin and by insulin resistance. It is still a debatable issue among investigators whether the initial stage in the pathogenesis of the disease consists in insulin deficiency or in insulin resistance. Generally speaking, insulin resistance is considered the primary defect in type 2 diabetes, constituting the link between the disease and obesity. According to a hypothesis developed mainly over the past few years and based on clamp techniques, insulin resistance is a defect of beta cells caused by an increase in the release of fatty acids and by overexposure of tissues to the latter.

Diabetes is associated with major clinical complications, including macrovascular damage (arteriosclerosis, myocardial infarct, peripheral arterial damage) and microvascular damage (retinopathy, nephropathy and neuropathy). The late complications of diabetes are an inevitable characteristic of the disease and constitute a serious threat to the well-being and normal quality of life of the individual.

The risk of coronary artery damage, which is the cause of death in 30-40% of patients above the age of 40, is three times greater in men and women with diabetes than in normal subjects *(UK Prospective Diabetes Study, UKPDS 1976-1997).*

### State of the art

The primary aim of antidiabetic therapy is to reduce hyperglycaemia levels and control glycaemia.

As things stand at present, the drugs available on the market are old drugs such as metformin and the sulphonylureas and recently introduced drugs such as the α-glucosidase inhibitors and thiazolinediones. As is understandable, the strategies aimed at controlling glucose homeostasis in type 2 diabetes differ and correspond to the different abnormalities presented by the diabetes.

The sulphonylureas promote the secretion of insulin by the beta cells and are capable of increasing the acute release of insulin, which is deficient in type 2 diabetes, and therefore of improving the control of post-prandial glucose.

Hypoglycaemia is the most common side effect of the sulphonylureas and may be severe and long-lasting. The sulphonylureas, moreover, may impede the vasodilatation of the heart in the case of ischaemia and may sometimes give rise to arrhythmias.

The biguanides came onto the market in the '50s, the best known of these drugs being metformin, which will be discussed in detail here below, in the context of the technical problem solved by the present invention.

The α-glucosidase inhibitors such as acarbose and voglibose aim at solving the problem of post-prandial hyperglycaemia by slowing down the absorption of carbohydrates in the bowel. These substances are competitive inhibitors of gastrointestinal α-glucosidase, an enzyme that splits starch and sucrose into monosaccharides. Like metformin, and unlike the sulphonylureas, they do not cause hypoglycaemia and weight gain. The α-glucosidase inhibitors require dosage adjustments for individual patients and the dose has to be high enough to slow down digestion in the small bowel, but also low enough to ensure that digestion is complete prior to entry of carbohydrates into the large bowel (to avoid intestinal side effects). The main side effect reported is flatulence (19%), followed by diarrhoea (3.8%). The α-glucosidase inhibitors do not relieve the liver production of glucose which is active far from mealtimes, and in the postabsorption and fasting conditions.

The thiazolidinediones (troglitazone, pioglitazone and rosiglitazone) are oral serum-glucose-lowering agents which have recently come onto the market with very considerable success. In 1998, the turnover of troglitazone (Rezulin) in the Unites States was 748 million dollars, which is only slightly lower than the 861 million dollar turnover of metformin (Glucophage), the top-ranking oral antidiabetic agent on the American market. The thiazolidinediones increase the insulin sensitivity of the tissues. They are capable of reducing hyperglycaemia and partly diabetic hyperlipidaemia as well as of reducing insulin levels. It has been observed that these compounds are agonists of PPARγ, a nuclear receptor which is present above all in adipose tissue, and induce the transcription of genes controlled by insulin, such as the lipoprotein lipase (LPL) gene. The serum-glucose-lowering effect of the compounds is perhaps secondary to their activity in recruiting fatty acids and triglycerides from the bloodstream and to the production of adipocytokines, without, however, our being able to rule out the possibility that the compounds may have a direct action on muscle tissue. The compounds, in fact, are capable of improving the uptake and transportation of glucose in muscle *in vivo* and *in* vitro. The most feared side effect of these compounds is liver damage even to the extent of liver failure. The use of these compounds also gives rise to an increase in weight, fluid retention, anaemia due to dilution of plasma volume, and various other side effects.

Thus, the sulphonylureas, biguanides, α-glucosidase inhibitors and thiazolidinediones are classes of drugs on the market today for the control of glucose in type 2 diabetes.

It is clear to experts in the field that, despite the very substantial research effort to come up with effective therapies for diabetes, no active ingredient is yet available which is capable of preventing the long-term evolution of the disease. Thus, given the present state of our knowledge, any monotherapy is destined to fail, making combination therapy necessary. The combination is sometimes required right from the outset in the absence of an adequate response to monotherapy.

As regards metformin, this was introduced in Europe in the '50s and in the United States in 1994 and is widely used in the treatment of type 2 diabetes and is the drug of choice in the therapy of type 2 diabetes associated with obesity. Metformin reduces the intestinal absorption of glucose, its production by the liver *(Cusi and De Fronzo, Diabetes Rev., 6: 89-131, 1998; Hundal et al., Diabetes, 49: 2063-2069, 2000)* and enhances its transportation and use in tissues, promoting its uptake in muscle, when stimulated by insulin (*Galuska et al., Diabetologia, 37:, 826-832, 1994; Bailey et al., New England J. Med., 334: 574-579, 1996).* Its action is also related to lipid metabolism through a reduction in levels of fatty acids and triglycerides in the blood *Cusi et al., J. Clin. Endocrinol. Metab., 81: 4059-4067, 1996; Kim et al., Diabetes, 51: 443-448, 2002).* Metformin, moreover, is thought to be capable of restoring normal insulin secretion altered by chronic exposure to fatty acids and to high levels of glucose *(Patanè et al,. Diabetes, 49: 735-740, 2000)* and of inhibiting adipose tissue lipase stimulated by catecholamines *(Flechtner-Mors et al., Diabetes Med., 16: 1000-1006, 1999).* There has to date been no satisfactory explanation of the mechanism of its serum-glucose-lowering action and the molecular action sites of metformin are still largely unclear *(Wiernsperger and Bailey, Drug, 58: 31-39, 1999; Hundal et al,. Diabetes, 49: 2063-2069, 2000 ; Musi et al., Diabetes, 51: 2074-2081, 2002; Hawley et al, Diabetes, 51: 2420-2425, 2002).* It would appear that the reduction of liver production of glucose by metformin is related to a decrease in the levels of key enzymes in glucogenesis such as glucose-6-phosphatase, phosphoenolpyruvate carboxy-kinase (PEPCK), and fructose-1,6-biphosphatase *(Fulgencio et al., Biochem. Pharmacol., 62:* 439-446, *2001; Song et al., Am. J. Physiol. Endocrinol. Metab., 281: E275-E282, 2001)* and is partly mediated by suppression of fatty acid oxidation *(Perriello et al., Diabetes, 43: 920-928, 1994).* It has, however, been proved that, apart from the gaps in our knowledge of its mechanisms and processes, metformin is capable of improving the use of glucose and the lipid profile, thereby reducing insulin resistance *(Bailey, Diabetes Care, 15: 755-742, 1992; Cusi and De Fronzo, Diabetes Rev., 6: 89-131, 1998).* This is what also emerges from a recent comparison between metformin and the modern thiazolidinediones *(Kim et al., Diabetes, 51: 443-448, 2002; Ciaraldi et al., 51: 30-36, Diabetes, 2002).* By improving the lipid profile, metformin consequently reduces the cardiovascular risk, particularly the incidence of myocardial infarct, as demonstrated by the UKPDS comparing metformin with the sulphonylureas and insulin *(UKPDS Group, Lancet 352: 837-853, 1998),* and also reduces the overall mortality in obese diabetic patients *(O'Connor et al., J. Fam. Fract. 47 Suppl5: S13-S22, 1998)*. This aspect, which is related to the improvement in the lipid profile, is essential, considering that dyslipidaemia in diabetes increases the risk of cardiovascular damage, which affects more than 50% of diabetic patients *(Wilson et al., Drug Aging, 58: 248-251, 2001).* Metformin reduces hyperglycaemia by 20% (range: 13-37%), when used as monotherapy after the failure of diet and physical exercise *(UKPDS II, Diabetes, 34: 793-798, 1985; De Fronzo et al., J. Clin. Endocrinol. Metab., 73: 1294-1301, 1991; Ciaraldi et al., Diabetes, 51: 30-36, 2002),* and by 25% in combination with sulphonylureas *(Reaven et al., J. Clin. Endocrinol. Metab., 74: 1020-1026, 1992).*

The action of metformin, however, presents certain limitations and therefore leaves room for improvement. Metformin therapy is limited by the decline in the duration of its efficacy *(Guay, Pharmacotherapy, 18: 1095-1204, 1998; Riddle, Am. J. Med., 108 Suppl. 6a: S15-S22, 2000; Charpentier, Diab. Metab. Res. Rev., 18 Suppl. 3: S70-S76, 2002).*

This problem is also accompanied by lactic acidosis, which constitutes the most important side effect. In addition, the patient may develop diarrhoea, nausea and gastrointestinal disorders, which have a high incidence (approximately 20%) and reduce patient acceptance of the drug.

Conditions of renal distress, liver dysfunction and cardiac insufficiency increase the risk of lactic acidosis *(Krentz, Drug Saf., 11: 223-241, 1994),* owing to the danger of an excessive increase in metformin levels in the body *(Jennings, Drug Aging, 10: 323-331, 1997).*

Metformin therapy, however, is of fundamental importance though the questions of its progressive failure in the monotherapy regimen and its side effects have still to be solved.

R-4-trimethylammonio-3-(tetradecylcarbamoyl)-amino-butyrate (hereinafter also referred to as ST1326) is a reversible, selective inhibitor of hepatic CPT1, capable of reducing the impact that fatty acid oxidation has on gluconeogenesis. Fatty acid oxidation favours the process of gluconeogenesis, by stimulating pyruvic carboxylase with an increase in acetylCoa levels and facilitating the cytosolic processes of glucose synthesis with an abundance of ATP and a high NADH/NAD⁺ ratio. This compound, disclosed in WO 99/59957, filed in the name of the present applicant, differs from irreversible, non-selective inhibitors such as etomoxir which have been found to cause cardiac hypertrophy *(Lesniak et al., Mol. Cell. Biochem., 142: 25-34, 1995; Vetter et al., Eur. Heart. J., 16 Suppl C: 15-19, 1995).* The antidiabetic activity of the compound, related to the oxidation of fatty acids, is particularly relevant to the postabsorption and fasting period..

The hepatic production of glucose, which is abnormal right from the start of the pathogenesis of diabetes *(Osei, Diabetes, 39: 597-601, 1990)* increases substantially during the progression of the disease, fuelling the hyperglycaemia of the patient with frank diabetes *(De Fronzo et al., Diabetes Care, 15: 318-368, 1992).* The hyperglycaemia, in turn, is sustained by increased oxidation of fatty acids *(Golay et al., Metabolism, 36: 692-696, 1987)* and facilitated by the drop in insulin levels and by hepatic insulin resistance *(De Fronzo et al., Metabolism, 338: 387-395, 1989).*

The reason for adopting the drug combination in diabetes therapy as outlined above is, in the first place, the complexity of the disease, which involves several processes and organs; perhaps this is precisely why, in a certain percentage of patients, satisfactory control of the disease cannot be achieved with the drugs currently available *(Rutten, Ned. Tijdschr. Geneeskd., 145: 1547-1550, 2001).* In diabetic patients, moreover, oral monotherapy which initially seems effective is unfortunately associated with a high secondary failure rate *(Brown et al., Clin. Ther., 21: 1678-1687, 1999; Riddle, Am. J. Med., 108 Suppl. 6a: S15-S22, 2000).* In addition, primary failure is by no means unusual in patients with high blood glucose levels at the start of monotherapy *(Charpentier, Diabetes Metab. Res. Rev., 18 Suppl3: S70-S76, 2002).* Thus it is that the idea of using a combination of drugs right from the start of pharmacological treatment is steadily gaining ground *(Charpentier, Diab. Metab. Res. Rev., 18 Suppl3: S70-S76, 2002).*

Even today, however, after the failure of diet and physical exercise, the treatment of diabetes almost invariably begins with monotherapy and only at a later stage, when the control of glycaemia is poor and monotherapy inadequate, is a drug combination resorted to, with the addition of a second and sometimes a third drug, e.g. metformin and sulphonylureas *(Riddle, Am. J. Med., 108 Suppl. 6a: S15-S22, 2000),* meglitinide and/or thiazolidinediones and metformin, and other types of combinations.

The use of the combination is motivated not only by the complexity of the disease and the failure of monotherapy, but also by the quest for greater efficacy (an additive and/or synergic effect) and by the possibility of reducing the side effects as a result of using lower doses.

Experts in the field are familiar with the fact that a synergic effect is not predictable *a priori.*

### Summary of the invention

It has now surprisingly been found that the combination of R-4-trimethylammonio-3-(tetradecylcarbamoyl)-amino-butyrate (ST1326) and metformin effectively succeeds in improving the control of glycaemia over the 24-hour period, especially far from mealtimes, in the postabsorption condition and in the fasting condition.

Therefore, one object of the present invention is a combination of R-4-trimethylammonio-3-(tetradecylcarbamoyl)-aminobutyrate or one of its pharmaceutically acceptable salts and metformin or one of its pharmaceutically acceptable salts, as well as the use of said combination as an antidiabetic medicament, particularly for the preparation of an antidiabetic drug for the treatment of type 2 diabetes. Said drug is useful for the control of glycaemia over the 24-hour period, particularly in the period far from mealtimes, and particularly during the night, in the postabsorption period and in fasting conditions. A further object of the present invention is to provide a medicament for the treatment of diabetes which presents no or substantially reduced side effects and, moreover, which can be used in patients for whom metformin is contraindicated or inadvisable, for example, those suffering from, or at risk of, one or more complications belonging to the group consisting of kidney damage, cardiac insufficiency, chronic liver damage, clinical proteinuria, peripheral vascular damage or lung damage.

A further object of the present invention is a pharmaceutical composition containing said combination.

The combination according to the present invention has shown a surprising synergic effect which makes it possible to provide effective antidiabetic therapy using subpharmacological doses of the respective components, with obvious advantages for the patient in terms of side effects, of which the combination is substantially devoid or which it presents only in a reduced form. If desired, the use of the combination containing pharmacological doses of R-4-trimethylammonio-3-(tetradecylcarbamoyl)-aminobutyrate or one of its pharmaceutically acceptable salts and subpharmacological doses of metformin or one of its pharmaceutically acceptable salts, or vice versa, or pharmacological doses of both, is envisaged and, in any case, possible in the present invention.

The above-mentioned WO 99/59957 discloses the compound ST1326 and its pharmaceutically acceptable salts, as well as its preparation and pharmacological activity. In this reference, the possibility of combining ST1326 with other antidiabetic drugs, including metformin, is generically mentioned, but there is not the slightest reference to the technical problem addressed and solved in the present invention, and, particularly, no mention is made either of the reduction or disappearance of the side effects of metformin, or, even less, to the synergic effect of the combination or even to the improvement in overall therapeutic cover in the diabetic patient.

The present invention will now be disclosed in detail, also by means of examples.

### Detailed description of the invention.

In the light of the considerations outlined above, the combination caters for the need to have to resort to the use of a second drug when monotherapy proves unsuccessful.

The use of the combination of ST1326 and metformin is supported by the increased activity that is exerted according to a synergic mechanism, as demonstrated in the examples given here below. Both compounds, in fact, are involved in gluconeogenesis, but in relation to different processes and enzymatic activities: in the case of metformin, the targets would appear to be the enzymes of gluconeogenesis, whereas in the case of ST1326 the target is the CPT1 of the fatty acid oxidation and transport system. This different actions made it impossible to predict the synergic effect of the combination according to the present invention, particularly in terms of dosages.

However much the inventors of the present invention may wish not to be constrained by any particular theory, from an analysis of the results one might postulate *a posteriori* that the synergism is probably due to that aspect of the action of metformin that has to do with insulin sensitivity and that it is related to improvement in the lipid profile. It is possible, in fact, that the activity of ST1326, aimed at reducing fatty acid oxidation and at reducing in this way the production of glucose by the liver, is conditioned and counteracted by counter-regulatory hormones (glucagon, GH, corticosterone) which operate in favour of lipolysis and gluconeogenesis *(Fery et al., Am. J. Physiol., 270: E822-E830, 1996).* The counterregulatory activity, however, is, in turn, conditioned by insulin, and thus by insulin sensitivity: all in all, then, the greater insulin sensitivity deriving from the action of metformin "enables" the activity of ST1326 to express itself in the combination with effects superior to those of ST1326 alone. This hypothesis, however, could not be formulated on the basis of the knowledge available to the expert in the field. In other words, the inventors of the present invention have observed that, as a result of the improvement in insulin sensitivity, the counterregulatory response is partly repressed, and the inhibitory action of ST1326 on fatty acid oxidation and gluconeogenesis is strengthened, since it is no longer competitively counteracted.

The synergic effect therefore makes it possible to use lower doses and consequently the side effects of the individual components can be reduced or eliminated.

In particular, by lowering the dose of metformin, the conditions in which it is contraindicated or which call for caution with its use, such as kidney damage, cardiac insufficiency, chronic liver damage, clinical proteinuria, peripheral vascular damage, lung damage, etc., may take on less importance.

The incidence of these disorders is far from rare. According to a retrospective analysis of a study of patients in a university diabetes clinic in the U.K., the incidence of conditions regarded as contraindications or risk factors for the use of metformin may be as much as 54% *(Sulkin et al., Diabetes Care, 20: 925-928, 1997).*

In addition, a retrospective study of patients in Scotland using metformin over the period from January 1993 to June 1995, the use of the drug was contraindicated in 24.5% *(Emslie-Smith et al., Diabet Med, 18: 483-488, 2001).*

Lastly, in an analysis of 306 patients treated with metformin, first at home and then in a general hospital in Germany, over the period from January 1995 to May 1998, it was found that, as a result of acute damage or of optimisation of treatment, 73% of the patients at admission presented contraindications, risk factors or concomitant diseases requiring the discontinuation of metformin *(Holstein et al., Diabet. Med., 16: 692- 696, 1999).*

In conclusion, then, though lactic acidosis is still a rare event *(Howlett and Bailey, Drug. Saf., 20: 489-503, 1999),* lowering the treatment dose by combining metformin with ST1326 might reduce the importance of those conditions in which the use of metformin is contraindicated or calls for caution, which are considerable during the progression of the disease

Similar advantages in terms of reduced side effects can be achieved for the same reasons as compared to the use of monotherapy with ST1326 alone.

The potential adverse effects of ST1326 alone, which may currently be postulated on the basis of the effects of the compound in rats and mice, may consist in an increase in fatty acids in the blood and lipid deposits in the liver.

The data obtained in monkeys, however, suggest that such effects may be related to the rodent metabolism model and may therefore not be reproducible in the same way in other species closer to man.

As regards the aspects relating to industrial applicability, the combination according to the present invention may be formulated conventionally in a pharmaceutical composition. This composition can be a simple combination of known pharmaceutical forms of the individual active ingredients, the dosage of which will be established according to modalities deriving from the application of the teachings of the present invention, that is to say, reduced doses such as to ensure the reciprocal synergism and, if so desired, the reduction or disappearance of side effects, particularly those of metformin. In this case, the composition according to the present invention can also be in the form of a kit, i.e. a pack containing the individual dosage forms of the active ingredients and the instructions for their simultaneous or sequential administration. Alternatively, the present invention provides for a new pharmaceutical composition containing the two active ingredients in a single dosage form. Advantageously, this dosage form will contain effective amounts of the active ingredients such as to provide therapeutic cover with a minimum number of administrations per day. In one preferred realisation of the invention, the pharmaceutical composition will also contain a dosage unit such as to ensure therapeutic cover overnight.

The doses and administration modalities will be established by the expert in the field, for example by the clinician or primary care physician, on the basis of his or her general knowledge and expertise. One preferred example of a dosage envisaged is a dose of R-4-trimethylammonio-3-(tetradecylcarbamoyl)-aminobutyrate ranging from 10 mg to 1 g or an equivalent dose of one of its pharmaceutically acceptable salts and a metformin dose ranging from 50 mg to 2.5 g or an equivalent dose of one of its pharmaceutically acceptable salts.

The pharmaceutical compositions according to the present invention are entirely conventional and do not require any particular description. In any event, a description of pharmaceutical compositions is to be found in the above-mentioned WO 99/59957 and, in greater detail, in Remington's Pharmaceutical Sciences, latest edition, Mack Publishing and Co.

The following example further illustrates the invention.

### Example

### Synergistic serum-glucose-lowering activity of ST1326 and metformin in the ob/ob mouse, the db/db mouse and the C57BL/6 mouse on a high-fat diet.

Mutations in laboratory animals have enabled models to be developed presenting non-insulin-dependent diabetes associated with obesity, hyperliperlipidaemia and insulin resistance and which allow the efficacy of new antidiabetic compounds to be tested *(Reed and Scribner, Diabetes, Obesity and Metabolism, 1: 75- 86, 1999).*

Extensively used models of genetically diabetic mice are the ob/ob mouse and db/db mouse models. The genetic basis of these models is a defect in the leptin gene (ob/ob mouse) or in the leptin receptor gene (db/db mouse), which causes leptin resistance and leads to hyperphagia, obesity, hyperinsulinaemia and insulin resistance, with hyperglycaemia as a result *(Hummel et al., Science 153: 1127-1128, 1996; Coleman, Diabetologia 14: 141-148, 1978; Kodama et al., Diabetologia 37: 739 - 744, 1994; Zhang et al., Nature 372: 425-432, 1994; Halaas et al., Science 269: 543-546, 1995; Chen et al., Cell 84: 491 - 495, 1996).*

Since hyperglycaemia is accompanied by obesity and insulin resistance, ob/ob and db/db mice have characteristics resembling those of type 2 diabetes in human subjects.

In addition to the genetic models of diabetes, other frequently used models are those including environmental factors such as the type of diet, owing to their importance in the pathogenesis of diabetes.

An example is the C57BL/6 mouse, which, as a result of exposure to a high-fat diet, develops not only insulin resistance but also mild hyperglycaemia *(Surwit et al., 37: 1163-1167, 1988; Luo et al., Metabolism 47*: *663-668, 1998).*

The B6. V-Lep ob/ob, C57BL/KsJ db/db and C57BL/6 mice used in the experiments were supplied by Jackson Lab. (via Charles River).

The ST1326 dose to be used in the ST1326-metformin combination, was obtained from previous experiments, which indicated the dose of 100 mg/kg/day as effective and the dose of 40 mg/kg/day as inactive in lowering serum glucose in db/db mice (Table 1).

The metformin dose to be used was obtained from the literature *(Meglasson et al., J. Pharmacol. Exp. Ther. 266: 1454-1462, 1993)* which indicated the dose of 900 mg/kg/day as effective in reducing hyperglycaemia by 50% in the KKAy mouse, a model of genetic, obese, hyperinsulinaemic and hyperglycaemic diabetes similar to the db/db and ob/ob mouse models, whereas the 200 mg/kg/day dose was ineffective, as well as from previous experiments of ours indicating the 600 mg/kg/day dose (Table 2) as effective in reducing hyperglycaemia by 22% in the ob/ob mouse in the Insulin Suppression Test (IST), whereas it proved ineffective at the dose of 200 mg/kg/day.

Blood samples were taken from ob/ob and db/db mice in standard environmental conditions, on a normal diet (4 RF21, Mucedola) and from C57BL/6J mice on a high-fat diet (D-12331, Research Diets) for 3 months, in postabsorption conditions (fasting from 8.30 a.m. to 4.30 p.m.) from the caudal vein, with the aid of a Jelco 22G catheter (Johnson and Johnson).

The blood was subjected to glucose level tests for the purposes of a well-matched distribution of the mice in the treatment groups.

At the start of treatment the body weights of the mice were measured, and monitoring of the consumption of water and animal feed was scheduled.

The mice were treated orally twice daily (at 8.30 a.m. and 6.30 p.m.) with ST1326 at the dose of 30 mg/kg and with metformin at the dose of 200 mg/kg, alone or in combination.

Blood samples were taken on various different days after administration of the products and in different nutritional conditions for the serum glucose determinations, OGTT (Oral Glucose Tolerance Test) and IST (Insulin Suppression Test: *Fujita et al., Diabetes 32: 804-810, 1983; Meglasson et al., J. Pharm. Exp. Therap. 266: 1454-1462, 1993; Luo et al., J. Pharm. Exp. Therap. 288: 529-534, 1999*)*.*

In the OGTT, blood samples were taken 0, 30, 60 and 120 minutes after glucose loading (3 g/kg).

The results obtained revealed that the single components exerted no significant serum-glucose-lowering activity at the experimental doses used, whereas their use in combination proved capable of significantly lowering hyperglycaemia with a synergic action. (Tables 3-8).

In the IST, blood samples were taken 60 min after subcutaneous loading of glucose (3 g/kg), insulin (Humulin R., Lilly 2 U/kg) and so-matostatin (0.5 mg/kg).

**Table 1**

| DB/DB MOUSE | | |
|---|---|---|
| Serum glucose levels (mg/dl) of male db/db mice treated with ST1326 or vehicle (deionised H₂O) at oral doses of 20 and 50 mg/kg twice daily (8.30 a.m. and 5.30 p.m.) for 45 days, in postabsorption conditions (fasting from 8.00 a.m. to 5.30 p.m.) and 8 hours after the last treatment (mean values ± s.e.) | | |
| Group | Dose mg/kg | Glucose (mg/dl) db/db |
| CONTROL | -- | 576 ± 43 |
| ST1326 | 20 | 562 ± 40 |
| ST1326 | 50 | 350 ± 48 Δ |

| | | |
|---|---|---|
| Number of cases per group: 8. Student's t-test: Δ indicates *P* < 0.01 vs control. group. | | |

**Table 2**

| OB/OB MOUSE | | |
|---|---|---|
| Blood glucose levels (mg/dl) of male ob/ob mice treated orally with metformin 300 mg/kg or vehicle (deionised water H₂O) twice daily (8.30 a.m. and 6.30 p.m.) for 8 days, in the IST, in postabsorption conditions (fasting from 8.30 a.m. to 4.30 p.m.) and 8 hours after the last treatment (mean values ± s.e.) | | |
| Group | Dose mg/kg | Glucose mg/dl |
| CONTROL | --- | 527.4 ± 33.0 |
| METFORMIN | 300 | 412.1 ± 3.5 □ |

| | | |
|---|---|---|
| Number of case per group: 6. Student's t-test: □ indicates *P* < 0.05 vs control group. | | |

**Table 3**

| OB/OB MOUSE | | |
|---|---|---|
| Blood glucose levels (mg/dl) of male ob/ob mice treated with ST1326 (30 mg/kg) and metformin (200 mg/kg), alone or in combination, twice daily for 11 days, in feeding conditions and 15 hours after the last treatment (mean values ± s.e.) | | |
| Group | Dose mg/kg | Glucose mg/dl |
| CONTROL | -- | 394.1 ± 32.9 |
| ST1326 | 30 | 394.8 ± 25.7 |
| METFORMIN | 200 | 376.3 ± 20-4 |
| ST1326 + METFORMIN | 30 + 200 | 287.3 ± 18.7 □ |

| | | |
|---|---|---|
| Number of cases per group: 6. Student's t-test: ■ indicates *P* < 0.02 vs control group. | | |

**Table 4**

| Blood glucose levels (mg/dl) of male ob/ob mice treated with ST1326 (30 mg/kg) and metformin (200 mg/kg), alone or in combination, twice daily for 18 days, in the IST, in postabsorption conditions (fasting from 8.30 a.m. to 4.30 p.m.) and 8 hours after the last treatment (mean values ± s.e.) | | |
|---|---|---|
| Group | Dose Mg/kg | Glucose mg/dl |
| CONTROL | -- | 517,7 ± 48.7 |
| ST1326 | 30 | 524.2 ± 56.2 |
| METFORMIN | 200 | 433.7 ± 34.8 |
| ST1326 + METFORMIN | 30 + 200 | 362.7 ± 39.2 □ |

| | | |
|---|---|---|
| number of cases per group: 6. Student's t-test: □ indicates *P* < 0.05 vs control group. | | |

**Table 5**

| DB/DB MOUSE | | |
|---|---|---|
| Blood glucose levels (mg/dl) of male db/db mice treated with ST1326 (30 mg/kg) and metformin (200 mg/kg), alone or in combination, twice daily for 16 days, in postabsorption conditions (fasting from 9.00 a.m. to 3.00 p.m.) and 6 hours after the last treatment (mean values ± s.e.). | | |
| Group | Dose mg/kg | Glucose mg/dl |
| CONTROL | -- | 360.2 ± 20.2 |
| ST1326 | 30 | 370.7 ± 30.0 |
| METFORMIN | 200 | 337.8 ± 33.1 |
| ST1326 + METFORMIN | 30 + 200 | 269.9 ± 26.9 □ |

| | | |
|---|---|---|
| number of cases per group: 6. Student's t-test: ■ indicates *P* < 0.02 vs control group. | | |

**Table 6**

| Blood glucose levels (mg/dl) of male db/db treated with ST1326 (30 mg/kg) and metformin (200 mg/kg), alone or in combination, twice daily for 37 days, in the OGTT, in fasting conditions (overnight) and 15 hours after the last treatment (mean values ± s.e.) | | | | | | |
|---|---|---|---|---|---|---|
| Group | Dose mg/kg | Glucose mg/dl | | | | AUC |
| | | 0 min | 30 min | 60 min | 120 min | |
| CONTROL | -- | 247.2 ± 30.4 | 577.8 ± 24.5 | 503.0 ± 34.8 | 361.0 ± 40.7 | 54506 ± 3760 |
| ST1326 | 30 | 175.3 ± 25.6 | 557.2 ± 30.6 | 498.3 ± 23.5 | 284.6 ± 20.8 | 50308 ± 2430 |
| METFORMIN | 200 | 195.5 ± 21.5 | 530.9 ± 34.6 | 456.8 ± 52.4 | 344.4 ± 40.9 | 49746 ± 4720 |
| ST1326 + METFOR. | 30 + 200 | 58.4 ± 19.9 ▲ | 255.4 ± 81.4 Δ | 332.4 ± 84.9 | 256.1 ± 39.8 | 31180 ± 7557 ■ |

| | | | | | | |
|---|---|---|---|---|---|---|
| number of cases per group: 6. Student's t-test: ■, Δ and ▲ indicate P < 0.02, *P* < 0.01 and *P* < 0.001, respectively vs control group. | | | | | | |

**Table 7**

| C57BL/6J MOUSE ON HIGH-FAT DIET | | |
|---|---|---|
| Blood glucose levels (mg/dl) of male C57BL/6J mice on a high-fat diet treated with ST1326 (30 mg/kg) and metformin (200 mg/kg), alone or in combination, twice daily for 15 days, in postabsorption conditions (fasting from 9.00 a.m. to 3.00 p.m.) and 6 hours after the last treatment (mean values ± s.e.) | | |
| Group | Dose mg/kg | Glucose mg/dl |
| CONTROL on high-fat diet | -- | 168.5 ± 12.3 |
| ST1326 | 30 | 167.3 ± 4.7 |
| METFORMIN | 200 | 146.1 ± 9.2 |
| ST1326 + METFORMIN | 30 + 200 | 122.8 ± 4.7 Δ |
| CONTROL on standard diet | -- | 135.7 ± 6.5 □ |

| | | |
|---|---|---|
| Number of cases per group: 6. Student's t-test: □ and Δ indicate *P* < 0.05 and *P* < 0.01, respectively, vs control group on high-fat diet | | |

**Table 8**

| Blood glucose levels (mg/dl) of male C57BL/6 mice on a high-fat diet, treated with ST1326 (30 mg/kg) and metformin (200 mg/kg), alone or in combination, twice daily for 35 days, in the OGTT, in postabsorption conditions (fasting from 9.00 a.m. to 3.00 p.m.) and 6 hours after the last treatment (mean values ± s.e.) | | | | | | |
|---|---|---|---|---|---|---|
| Group | Dose mg/kg | Glucose (mg/dl) | | | | AUC |
| | | 0 min | 30 min | 60 min | 120 min | |
| high-fat diet | | 170.8 ± 12,4 | 259.7 ± 11.1 | 220.1 ± 6.9 | 210.0 ± 12.2 | 26554 ± 1018 |
| ST1326 | 30 | 156.0 ± 7.8 | 236.3 ± 11.2 | 207.0 ± 9.9 | 212.9 ± 8.6 | 25133 ± 652 |
| METFORMIN | 200 | 141.1 ± 11.4 | 241.2 ± 18.9 | 212.3 ± 5.0 | 186.2 ± 15.1 | 24487 ± 1182 |
| | | | | | | |
| ST1326 + METFOR. | 30+200 | 118.4 ± 6.3 Δ | 217.5 ± 5.6 Δ | 187.7 ± 2.9 Δ | 155.6 ± 6.9 Δ | 21414 ± 387 Δ |
| | | | | | | |
| CONTROL on | -- | 121.1 | 200.5 | 187.0 | 159.0 | 21016 ± 283 Δ |
| standard diet | | ± 4.4 Δ | ± 6.2 ▲ | ± 3.3 Δ | ± 4.1 Δ | |

| | | | | | | |
|---|---|---|---|---|---|---|
| Number of cases per group: 6. Student's t-test: Δ and Δ indicate *P* < 0.01 and *P* < 0.001, respectively, vs control group on high-fat diet. | | | | | | |

## Claims

1. Combination of R-4-trimethylammonio-3-(tetradecyl-carbamoyl)-aminobutyrate or one of its pharmaceutically acceptable salts and metformin or one of its pharmaceutically acceptable salts.

2. Use of the combination according to claim 1 as a medicament.

3. Use of the combination according to claim 1 for the preparation of a medicament for the treatment of type 2 diabetes.

4. Use according to claim 3 for the preparation of a medicament with antidiabetic activity for the control of glycaemia over the 24 hour period.

5. Use according to claim 4, where the medicament is useful for the control of glycaemia far from mealtimes, and in the postabsorption and fasting conditions.

6. Use according to any one of claims 2-5 for the preparation of a medicament with antidiabetic activity, said medicament being devoid of the side effects typical of the individual components of said combination or having only substantially reduced side effects of that type.

7. Use according to claim 6, where said medicament is used for the treatment of diabetic patients for whom metformin is contraindicated or inadvisable.

8. Use according to claim 6 or 7, where said medicament is indicated in patients suffering from one or more complications belonging to the group consisting of kidney damage, cardiac insufficiency, chronic liver damage, clinical proteinuria, peripheral vascular damage or lung damage.

9. Pharmaceutical composition containing the combination according to claim 1.

10. Pharmaceutical composition according to claim 9, containing subpharmacological doses of R-4-trimethyl-ammonio-3-(tetradecylcarbamoyl)-aminobutyrate or one of its pharmaceutically acceptable salts and of metformin or one of its pharmaceutically acceptable salts, respectively.

11. Pharmaceutical composition according to claim 9, containing pharmacological doses of R-4-trimethylammonio-3-(tetradecylcarbamoyl)-aminobutyrate or one of its pharmaceutically acceptable salts and subpharmaceutical doses of metformin or one of its pharmaceutically acceptable salts.

12. Pharmaceutical composition according to claim 9, containing subpharmacological doses of R-4-trimetyl-ammonio-3-(tetradecylcarbamoyl)-aminobutyrate or one of its pharmaceutically acceptable salts and pharmacological doses of metformin or one of its pharmacologically acceptable salts, respectively.

13. Pharmaceutical composition according to claim 9, containing pharmacological doses of R-4-trimetyl-ammonio-3-(tetradecylcarbamoyl)-aminobutyrate or one of its pharmaceutically acceptable salts and pharmacological doses of metformin or one of its pharmacologically acceptable salts, respectively.

14. Pharmaceutical composition according to any one of claims 9-13, containing R-4-trimethylammonio-3-(tetradecyl-carbamoyl)-aminobutyrate or one of its pharmaceutically acceptable salts and metformin or one of its pharmaceutically acceptable salts in a single dosage form.

15. Pharmaceutical composition according to claim 14, where one dosage unit is suitable for the therapeutic coverage of the nocturnal fasting period.

16. Pharmaceutical composition according to claims 9-15, in which ST 1326 is present at a dose ranging from 10 mg to 1 g or an equivalent dose of one of its pharmaceutically acceptable salts, and metformin is present at a dose ranging from 50 mg to 2.5 g or an equivalent dose of one of its pharmaceutically equivalent salts.

## Patentansprüche

1. Kombination von R-4-Trimethylammonium-3-(tetradecylcarbamoyl)-aminobutyrat oder eines dessen pharmazeutisch annehmbarer Salze und Metformin oder eines dessen pharmazeutisch annehmbarer Salze.

2. Verwendung der Kombination gemäß Anspruch 1, als Arzneimittel.

3. Verwendung der Kombination gemäß Anspruch 1, für die Herstellung eines Arzneimittels zur Behandlung von Typ-2-Diabetes.

4. Verwendung gemäß Anspruch 3, für die Herstellung eines Arzneimittels mit antidiabetischer Wirkung zur Kontrolle der Glykämie während eines 24-Stunden Zeitraums.

5. Verwendung gemäß Anspruch 4, wobei das Arzneimittel für die Kontrolle von Glykämie fern von Mahlzeiten verwendbar ist, und in der Postabsorption und unter Fastenbedingungen.

6. Verwendung gemäß irgendeinem der Ansprüche 2-5, für die Herstellung eines Arzneimittels mit antidiabetischer Wirkung, wobei das Arzneimittel frei von Nebenwirkungen ist, welche typisch für die einzelnen Komponenten der Kombination sind oder nur wesentlich reduzierte Nebenwirkungen dieser Art hat.

7. Verwendung gemäß Anspruch 6, wobei das Arzneimittel für die Behandlung von diabetischen Patienten verwendet wird, für welche Metformin kontraindiziert oder nicht zu empfehlen ist.

8. Verwendung gemäß Anspruch 6 oder 7, wobei das Medikament bei Patienten angezeigt ist, welche an einer oder mehreren Komplikationen leiden, die ausgewählt sind aus der Gruppe bestehend aus Nierenschädigung, Herzinsuffizienz, chronische Leberschädigung, klinische Proteinurie, periphere Gefäßschädigung oder Lungenschädigung.

9. Pharmazeutische Zusammensetzung enthaltend die Kombination gemäß Anspruch 1.

10. Pharmazeutische Zusammensetzung gemäß Anspruch 9, enthaltend subpharmakologische Dosen von R-4-Trimethylammonium-3-(tetradecylcarbamoyl)-aminobutyrat oder eines dessen pharmazeutisch annehmbarer Salze und Metformin oder eines dessen pharmazeutisch annehmbarer Salze.

11. Pharmazeutische Zusammensetzung gemäß Anspruch 9, enthaltend pharmakologische Dosen von R-4-Trimethylammonium-3-(tetradecylcarbamoyl)-aminobutyrat oder eines dessen pharmazeutisch annehmbarer Salze und subpharmakologische Dosen von Metformin oder eines dessen pharmazeutisch annehmbarer Salze.

12. Pharmazeutische Zusammensetzung gemäß Anspruch 9, enthaltend subpharmakologische Dosen von R-4-Trimethylammonium-3-(tetradecylcarbamoyl)-aminobutyrat oder eines dessen pharmazeutisch annehmbarer Salze und pharmakologische Dosen von Metformin oder eines dessen pharmazeutisch annehmbarer Salze.

13. Pharmazeutische Zusammensetzung gemäß Anspruch 9, enthaltend pharmakologische Dosen von R-4-Trimethylammonium-3-(tetradecylcarbamoyl)-aminobutyrat oder eines dessen pharmazeutisch annehmbarer Salze und pharmakologische Dosen von Metformin oder eines dessen pharmazeutisch annehmbarer Salze.

14. Pharmazeutische Zusammensetzung gemäß irgendeinem der Ansprüche 9-13, enthaltend R-4-Trimethylammonium-3-(tetradecylcarbamoyl)-aminobutyrat oder eines dessen pharmazeutisch annehmbarer Salze und Metformin oder eines dessen pharmazeutisch annehmbarer Salze in einer Einzeldosisform.

15. Pharmazeutische Zusammensetzung gemäß Anspruch 14, wobei eine Dosiseinheit für die therapeutische Abdeckung der nächtlichen Fastenperiode geeignet ist.

16. Pharmazeutische Zusammensetzung gemäß den Ansprüchen 9-15, in welchen ST 1326 in einer Dosis vorliegt, welche sich von 10 mg bis 1 g oder einer äquivalenten Dosis eines dessen pharmazeutisch annehmbarer Salze erstreckt, und Metformin in einer Dosis vorliegt, welche sich von 50 mg bis 2,5 g oder einer äquivalenten Dosis eines dessen pharmazeutisch annehmbarer Salze erstreckt.

## Revendications

1. Combinaison de R-4-triméthylammonio-3-(tétradécylcarbamoyl)-aminobutyrate ou l'un de ses sels pharmaceutiquement acceptables et metformine ou un de ses sels pharmaceutiquement acceptables.

2. Utilisation de la combinaison selon la revendication 1, en tant que médicament.

3. Utilisation de la combinaison selon la revendication 1, pour la préparation d'un médicament pour le traitement du diabète de type 2.

4. Utilisation selon la revendication 3, pour la préparation d'un médicament présentant une activité antidiabétique pour la régulation de la glycémie sur la période de 24 heures.

5. Utilisation selon la revendication 4, dans laquelle le médicament est utile pour la régulation de la glycémie en dehors des repas, et dans les conditions de postabsorption et à jeun.

6. Utilisation selon l'une quelconque des revendications 2 à 5 pour la préparation d'un médicament présentant une activité antidiabétique, ledit médicament étant dépourvu d'effets secondaires typiques des composants individuels de ladite combinaison ou présentant des effets secondaires de ce type seulement sensiblement réduits.

7. Utilisation selon la revendication 6, dans laquelle ledit médicament est utilisé pour le traitement de patients diabétiques pour qui la metformine est contre indiquée ou déconseillée.

8. Utilisation selon la revendication 6 ou 7, dans laquelle ledit médicament est indiqué chez les patients souffrant d'une ou plusieurs complications appartenant au groupe constitué par une lésion rénale, une insuffisance cardiaque, une lésion du foie chronique, une protéinurie clinique, une lésion vasculaire périphérique ou une lésion pulmonaire.

9. Composition pharmaceutique contenant la combinaison selon la revendication 1.

10. Composition pharmaceutique selon la revendication 9, contenant des doses subpharmacologiques de R-4-triméthylammonio-3-(tétradécylcarbamoyl)-aminobutyrate ou un de ses sels pharmaceutiquement acceptables et de metformine ou un de ses sels pharmaceutiquement acceptables, respectivement.

11. Composition pharmaceutique selon la revendication 9, contenant des doses pharmacologiques de R-4-triméthylammonio-3-(tétradécylcarbamoyl)-aminobutyrate ou un de ses sels pharmaceutiquement acceptables et des doses subpharmaceutiques de metformine ou un de ses sels pharmaceutiquement acceptables.

12. Composition pharmaceutique selon la revendication 9, contenant des doses subpharmacologiques de R-4-triméthyl-ammonio-3-(tétradécylcarbamoyl)-aminobutyrate ou un de ses sels pharmaceutiquement acceptables et des doses pharmacologiques de metformine ou un de ses sels pharmacologiquement acceptables, respectivement.

13. Composition pharmaceutique selon la revendication 9, contenant des doses pharmacologiques de R-4- triméthylammonio-3-(tétradécylcarbamoyl)-aminobutyrate ou un de ses sels pharmaceutiquement acceptables et des doses pharmacologiques de metformine ou un de ses sels pharmacologiquement acceptables, respectivement.

14. Composition pharmaceutique selon l'une quelconque des revendications 9 à 13, contenant du R-4-triméthylammonio-3-(tétradécyl-carbamoyl)-aminobutyrate ou un de ses sels pharmaceutiquement acceptables et de la metformine ou un de ses sels pharmaceutiquement acceptables sous la forme d'un dosage unique.

15. Composition pharmaceutique selon la revendication 14, dans laquelle une unité de dosage est appropriée pour la couverture thérapeutique de la période de jeûne nocturne.

16. Composition pharmaceutique selon les revendications 9 à 15, dans laquelle du ST 1326 est présent à une dose allant de 10 mg à 1 g ou une dose équivalente d'un de ses sels pharmaceutiquement acceptables, et de la metformine est présente à une dose allant de 50 mg à 2,5 g ou une dose équivalente d'un de ses sels pharmaceutiquement équivalents.
